# FASCICULE DE BREVET EUROPEEN

(11) **EP 1 131 109 B1**
(45) Date de publication et mention de la délivrance du brevet: **26.03.2003**
(21) Numéro de dépôt: 99956059.2
(22) Date de dépôt: 16.11.1999
(51) Int. Cl.: A61L 11/00

(54) **PROCEDE ET APPAREIL DE TRAITEMENT DE DECHETS CONTAMINES**
VERFAHREN UND VORRICHTUNG ZUR BEHANDLUNG VON VERUNREINIGTEN ABFÄLLEN
METHOD AND APPARATUS FOR TREATING CONTAMINATED WASTE

(30) Priorité: 17.11.1998 FR 9814426
(43) Date de publication de la demande: 12.09.2001
(73) Titulaire: Medidest Patent Development Company, 9000 Funchal (PT)
(72) Inventeur: CEBOLLERO, Jean, F-64400 Oloron Sainte Marie (FR)
(74) Mandataire: Cabinet BARRE LAFORGUE & associés
(86) Numéro de dépôt international: FR9902806
(87) Numéro de publication internationale: WO00029040

(56) Documents cités:
- WO-A-93/06418
- DE-A- 19 722 109
- FR-A- 2 715 850

## Description

L'invention concerne le traitement des déchets contaminés tels que les déchets hospitaliers en vue de les transformer en déchets secs banals non contaminés. Dans tout le texte, le terme "contaminés" et ses dérivés désignent uniquement, de façon traditionnelle, une pollution d'origine biologique (germes pathogènes, virus, micro-organismes, contages ...).

Les déchets contaminés tels que les déchets hospitaliers peuvent être de nature très variée : il peut s'agir d'objets métalliques (aiguilles, seringues, ancillaires, prothèses, ...), et/ou en matières synthétiques (seringues, poches, emballages, pansements, ...), et/ou en matières fibreuses (tissus, cotons,...) et/ou de fluides (sang, ...) et/ou de compositions pharmaceutiques ou de nettoyage.... Cette variété complique considérablement le problème de leur traitement de transformation en déchets secs banals.

L'intérêt de réaliser un tel traitement des déchets contaminés avant leur mise en décharge, leur enfouissement ou leur incinération avec les déchets ménagers est reconnu. On peut classer les différentes solutions qui ont déjà été proposées pour réaliser ce traitement selon la nature du procédé de stérilisation retenu : les procédés chimiques ou par irradiation, qui sont complexes et coûteux, et les procédés par chauffage. Dans cette dernière catégorie, on distingue les procédés par chauffage à la vapeur sous pression et les procédés par chauffage à sec.

Les procédés de chauffage à la vapeur sous pression consistent à placer les déchets dans un autoclave en présence d'une pression de vapeur d'eau après d'éventuelles mises sous vide successives. Ces procédés constituent la voie privilégiée jusqu'à maintenant dans la mesure où la vapeur d'eau sous pression permet de stériliser les déchets rapidement (de l'ordre de 15mn), à relativement basse température (de l'ordre de 125°C), et de profiter des avantages de la présence de vapeur d'eau dans le milieu réactionnel (effet catalyseur de certaines réactions chimiques, homogénéisation de température au sein du milieu réactionnel, transfert thermique amélioré par la condensation de la vapeur d'eau). Néanmoins, ces procédés nécessitent une installation coûteuse (autoclave). En outre, les phases de chargement/mise sous pression et refroidissement/vidange sont longues et grèvent la productivité et la consommation énergétique. De surcroît, les déchets ainsi traités sont gorgés d'humidité et sont ultérieurement plus difficiles, et donc plus coûteux, à incinérer, et/ou présentent le risque de pouvoir produire des écoulements polluants.

Les propositions plus rares de stérilisation par chauffage à sec (FR-2715850 ou EP-400676) suivie d'un broyage présentent par contre l'inconvénient de ne pas garantir une stérilisation complète des déchets dans la mesure où certaines parties des déchets, isolées de l'intérieur, peuvent ne pas être chauffées. En outre, ces procédés nécessitent, pour un temps de traitement similaire, une température sensiblement plus élevée (de l'ordre de 200°C) que dans le traitement à la vapeur. Si le broyage intervient avant la stérilisation, le broyeur est alors contaminé et doit donc être stérilisé spécifiquement avant toute intervention de maintenance ou de réparation. Une telle stérilisation est coûteuse et difficile à réaliser. Elle peut être en pratique imparfaite, de sorte que les risques de contaminations ne sont pas nuls.

L'invention vise donc à pallier ces inconvénients en proposant un procédé et un appareil permettant d'effectuer le traitement des déchets contaminés tels que des déchets hospitaliers de façon économique (en investissements et/ou coûts d'utilisation), tout en assurant la stérilisation complète des déchets en une faible durée de traitement -notamment de l'ordre de ou inférieure à 15min-.

L'invention vise aussi à proposer un procédé et un appareil particulièrement sûrs et évitant toute contamination accidentelle des utilisateurs et du personnel effectuant les opérations de maintenance ou de réparation. L'invention vise aussi à permettre des interventions faciles de maintenance ou de réparation en cas de panne.

L'invention vise aussi à proposer un appareil simple, compact, robuste, et d'une grande fiabilité de fonctionnement.

L'invention vise aussi à proposer un procédé et un appareil de traitement n'émettant que peu de gaz résiduels et dans lesquels les gaz émis sont traités avant d'être rejetés dans l'atmosphère, notamment pour éviter les pollutions et mauvaises odeurs.

L'invention vise ainsi en particulier à proposer un appareil qui peut assurer de façon autonome le traitement des déchets à partir d'une simple alimentation en énergie électrique.

L'invention vise ainsi à proposer un procédé et un appareil pouvant être mis en oeuvre simplement sur un site de production des déchets -notamment en milieu hospitalier-.

Pour ce faire, l'invention concerne un procédé de traitement de déchets contaminés tels que des déchets hospitaliers dans lequel on traite les déchets dans une chambre de broyage/stérilisation définissant une enceinte close au sein de laquelle on broie les déchets et on les stérilise par chauffage, caractérisé en ce qu'on fait circuler les déchets dans la chambre de broyage/stérilisation en plusieurs cycles de broyage/stérilisation à travers des moyens de broyage. Ainsi, dans un procédé selon l'invention, les déchets sont broyés plusieurs fois et sont stérilisés par chauffage dans la chambre de broyage/stérilisation.

Avantageusement, un procédé selon l'invention est en outre caractérisé par au moins l'une des caractéristiques suivantes :
- à chaque cycle de broyage, on soumet les déchets à un chauffage dans la chambre de broyage/stérilisation à l'amont et/ou à l'aval et/ou au sein des moyens de broyage ;
- on stérilise les déchets par chauffage à sec -notamment par passage en regard de moyens de chauffage électrique et/ou en regard d'au moins un échangeur thermique-;
- on fait circuler les déchets en continu en cycles successifs sans interruption à travers les moyens de broyage entraînés en permanence et en regard de moyens de chauffage maintenus actifs en permanence ;
- on fait circuler les déchets en les récupérant en sortie des moyens de broyage sur des moyens de convoyage sans fin incorporés dans la chambre de broyage/stérilisation et adaptés pour réintroduire à l'entrée des moyens de broyage les déchets récupérés en sortie ; ces moyens de convoyage peuvent être formés d'un tapis transporteur sans fin à augets ; en variante, on fait circuler les déchets en les entraînant par un courant d'air pulsé à haute vitesse que l'on entretient au sein de la chambre de broyage/stérilisation depuis la sortie des moyens de broyage, et de bas en haut, dans au moins une cheminée disposée dans cette chambre ; dans cette variante, les déchets peuvent être stérilisés lors de leur descente vers les moyens de broyage ;
- pour chauffer les déchets, on place l'intérieur de la chambre de broyage/stérilisation à une température apte à stériliser les déchets qui s'y trouvent, on maintient les déchets dans la chambre de broyage/stérilisation pendant une durée adaptée pour assurer leur complète stérilisation ; les déchets sont ainsi broyés et chauffés dans la même chambre de broyage/stérilisation; on transfère ensuite les déchets dans une chambre de refroidissement séparée, puis on introduit de nouveaux déchets à traiter dans la chambre de broyage/stérilisation sans interruption des moyens de broyage ni du chauffage ;
- on évacue les gaz émis par le traitement, dits gaz de traitement, vers un poste de traitement des gaz avant leur rejet dans l'atmosphère ; on évacue les gaz de traitement hors de la chambre de broyage/stérilisation en permanence pendant toute la durée du traitement ; on chauffe les gaz dans le poste de traitement des gaz pour réaliser leur combustion catalytique, et on recycle les gaz chauds après leur traitement dans au moins un échangeur thermique à circuit de gaz séparé disposé dans la chambre de broyage/stérilisation; on maintient la chambre de broyage/stérilisation au moins sensiblement hermétiquement close pendant toute la durée du traitement, à l'exception, le cas échéant, de l'évacuation des gaz de traitement ; on maintient l'évacuation des gaz pendant le transfert des déchets vers la chambre de refroidissement et pendant une durée supplémentaire adaptée pour assurer l'évacuation de l'ensemble des gaz, qui peuvent être nocifs avant d'introduire de nouveaux déchets à traiter dans la chambre de broyage/stérilisation ;
- en cas de panne des moyens de broyage et/ou des moyens de chauffage et/ou des moyens de convoyage, on introduit dans la chambre de broyage/stérilisation une quantité d'eau qui est évaporée par chauffage dans la chambre de broyage stérilisation et/ou de vapeur d'eau, cette quantité étant apte à stériliser l'ensemble des déchets et autres éléments présents dans la chambre de broyage/stérilisation;
- on broie les déchets de façon à les diviser après plusieurs cycles de broyage en particules de moins de 10 mm de taille moyenne, de préférence de taille moyenne inférieure ou égale à 5 mm ;
- on chauffe les déchets à une température comprise entre 180°C et 220°C, notamment de l'ordre de 200°C ; on chauffe les déchets en maintenant cette température à l'intérieur de la chambre de broyage/stérilisation ;
- on transfère les déchets dans la chambre de refroidissement, et après refroidissement, on extrait puis on emballe des déchets traités, notamment dans un sac étanche tel qu'un sac d'ordures ménagères et on enregistre sur un support d'informations (impression d'une étiquette ou d'un code à lecture optique, enregistrement sur un support magnétique et/ou électronique, ...) des informations sur les déchets traités et/ou sur le traitement effectué.

L'invention s'étend à un appareil de traitement pour la mise en oeuvre d'un procédé selon l'invention.

L'invention concerne ainsi un appareil de traitement de déchets contaminés tels que des déchets hospitaliers, comprenant une chambre de broyage/stérilisation adaptée pour pouvoir définir une enceinte close renfermant des moyens de broyage des déchets et des moyens de chauffage adaptés pour pouvoir soumettre les déchets à un chauffage de façon à les stériliser, caractérisé en ce que la chambre de broyage/stérilisation comporte des moyens de convoyage adaptés pour pouvoir faire circuler les déchets en plusieurs cycles de broyage à travers les moyens de broyage.

Avantageusement et selon l'invention, un appareil selon l'invention est en outre caractérisé par au moins l'une des caractéristiques suivantes :
- les moyens de convoyage sont des moyens de convoyage sans fin adaptés pour récupérer les déchets en sortie des moyens de broyage et pour les réintroduire à l'entrée des moyens de broyage ;
- les moyens de convoyage sont adaptés pour transporter les déchets depuis la sortie des moyens de broyage, puis en regard des moyens de chauffage, puis à l'entrée des moyens de broyage ;
- les moyens de convoyage sont adaptés pour passer sous les moyens de broyage, et récupérer les déchets par gravité en sortie des moyens de broyage pour remonter les déchets au-dessus des moyens de broyage - notamment en regard de moyens de chauffage disposés dans la chambre de broyage/stérilisation plus haut que les moyens de broyage-, et pour laisser ensuite retomber les déchets à l'entrée des moyens de broyage par gravité ;
- les moyens de convoyage sont formés d'un tapis transporteur sans fin à augets passant autour et en dessous des moyens de broyage et en regard des moyens de chauffage ; en variante, les moyens de convoyage comportent des moyens pour créer et entretenir dans la chambre de broyage/stérilisation un courant d'air pulsé à haute vitesse passant depuis la sortie des moyens de broyage, puis circulant de bas en haut dans la chambre de broyage/stérilisation pour y entraîner les déchets ; cette dernière variante présente l'avantage d'éviter des parties mécaniques mobiles au sein de la chambre de broyage/stérilisation, et d'augmenter la vitesse de recirculation des déchets ; dans cette variante, les déchets peuvent être stérilisés en passant en regard des moyens de chauffage lors de leur descente vers les moyens de broyage ;
- dans la variante où ils sont formés d'un tapis transporteur, les moyens de convoyage sont adaptés pour définir :
   . un tronçon, dit tronçon inférieur, passant sous les moyens de broyage pour récupérer les déchets issus des moyens de broyage,
   . un tronçon, dit tronçon latéral remontant, passant en remontant en regard de moyens de chauffage pour soumettre les déchets à ces moyens de chauffage,
   . un tronçon, dit tronçon supérieur, passant au-dessus de l'entrée des moyens de broyage pour y décharger les déchets,
   . un tronçon, dit tronçon latéral descendant, prolongeant le tronçon supérieur vers le bas jusqu'au tronçon inférieur ;
   . les moyens de chauffage comportent des moyens de chauffage adaptés pour chauffer les déchets lors de leur descente et/ou pour renforcer la stérilisation des moyens de convoyage après décharge des déchets et avant récupération à nouveau de déchets ; avantageusement, dans la variante où les moyens de convoyage sont formés d'un tapis transporteur, le tronçon latéral descendant passe en regard de moyens de chauffage pour renforcer la stérilisation les moyens de convoyage après décharge des déchets et avant récupération à nouveau de déchets ;
- il comprend une chambre de refroidissement communiquant avec la chambre de broyage/stérilisation par une porte ; la chambre de refroidissement jouxte la chambre de broyage/stérilisation et la porte est articulée de façon à former une trappe qui, en position ouverte, s'étend en regard et sous une portion des moyens de convoyage à partir de laquelle les déchets chutent vers l'entrée des moyens de broyage, de sorte que cette porte capte les déchets avant leur réintroduction à l'entrée des moyens de broyage ; la chambre de refroidissement est close à l'exception d'au moins une entrée d'air de refroidissement et d'une évacuation des gaz ;
- il comprend un circuit d'évacuation des gaz comprenant un poste de traitement des gaz avant leur rejet dans l'atmosphère, qui peut être disposé dans la chambre de refroidissement dont il est isolé (pour empêcher toute fuite intempestive de gaz vers l'atmosphère de la chambre de refroidissement) ;
- le poste de traitement des gaz comprend des moyens de chauffage des gaz aptes à réaliser leur combustion catalytique ; au moins un échangeur thermique à circuit de gaz séparé est disposé dans la chambre de broyage/stérilisation, et relié au poste de traitement de gaz pour récupérer les gaz chauds après leur traitement, de sorte que cet échangeur thermique constitue un moyen de chauffage de la chambre de broyage/stérilisation et/ou des déchets en cours de traitement ; en variante, ces moyens de chauffage des gaz sont directement eux-mêmes incorporés dans la chambre de broyage/stérilisation ;
- la chambre de broyage/stérilisation est adaptée pour pouvoir être hermétiquement close pendant toute la durée de traitement, à l'exception, le cas échéant, de l'évacuation des gaz de traitement ; elle est équipée d'une soupape de sécurité limitant la valeur de la pression dans cette chambre ;
- les moyens de chauffage sont des moyens de chauffage à sec -notamment électrique et/ou à échangeur(s) de chaleur- ; les moyens de chauffage comprennent au moins un tunnel traversé par les moyens de convoyage et associé à une source de chaleur, telle que des résistances électriques et/ou un échangeur thermique à circuit de gaz séparé recevant les gaz issus du poste de traitement des gaz ;
- les moyens de broyage sont adaptés pour broyer les déchets de façon à les diviser après plusieurs cycles de broyage, en particules de moins de 10 mm de taille moyenne ;
- les moyens de chauffage sont adaptés pour maintenir dans la chambre de broyage/stérilisation une température comprise entre 180°C et 220°C -notamment de l'ordre de 200°C- ; il est à noter que cette température est particulièrement homogène au sein de la chambre de broyage/stérilisation ;
- il comprend une alimentation en eau et/ou vapeur d'eau dans la chambre de broyage/stérilisation permettant d'y introduire une quantité d'eau et/ou de vapeur d'eau apte à stériliser l'ensemble des déchets et autres éléments présents dans la chambre de broyage/stérilisation en cas de panne des moyens de broyage et/ou des moyens de chauffage et/ou des moyens de convoyage;
- il comprend des moyens d'emballage des déchets traités et des moyens d'impression d'une étiquette présentant des informations sur les déchets traités et/ou le traitement effectué ;
- il comprend un automate programmable de contrôle qui permet notamment de commander automatiquement le fonctionnement des moyens de broyage, et/ou des moyens de chauffage, et/ou des moyens de convoyage, et/ou des moyens d'évacuation des gaz, et/ou des portes mobiles, et/ou de l'alimentation en eau et/ou en vapeur d'eau en cas de panne.

L'invention permet de traiter des déchets contaminés de façon simple, rapide, sûre et économique. L'appareil selon l'invention est compact et peu coûteux à l'investissement et à l'utilisation et peut être installé sur le site même de production des déchets. Son fonctionnement est autonome et il ne nécessite aucune intervention de réglage ou de surveillance, à l'exception, le cas échéant, de la purge périodique des liquides résiduels du poste de traitement des gaz (cette purge étant même évitée si les gaz sont traités par post-combustion catalytique sous l'effet d'un chauffage intense). Les déchets sont stérilisés et broyés en une courte durée (classiquement de l'ordre de 15 min comme dans les traitements antérieurs à la vapeur sous pression, alors que les traitements antérieurs en chaleur sèche nécessitent une durée de l'ordre du double pour la stérilisation), sans aucune contamination résiduelle. En particulier, il a été constaté que le fait de broyer en plusieurs cycles et de stériliser les déchets concomitamment dans une même chambre close procure un traitement bien meilleur que dans l'art antérieur. Les déchets sont exempts d'humidité résiduelle après traitement et peuvent être aisément incinérés ou déposés en centre d'enfouissage ou en décharge contrôlée, comme de simples ordures ménagères. En outre, en cas de panne, il n'existe aucun risque de contamination puisque l'ensemble de la chambre de broyage/stérilisation peut être stérilisée avant intervention. Par ailleurs, il est aisé de nettoyer et stériliser entièrement l'appareil périodiquement en effectuant un traitement à vide (sans déchet) d'autonettoyage.

D'autres buts, caractéristiques et avantages de l'invention apparaissent de la description suivante de ses modes de réalisation préférentiels donnés à titre d'exemple non limitatif, et qui se réfère aux figures annexées dans lesquelles :

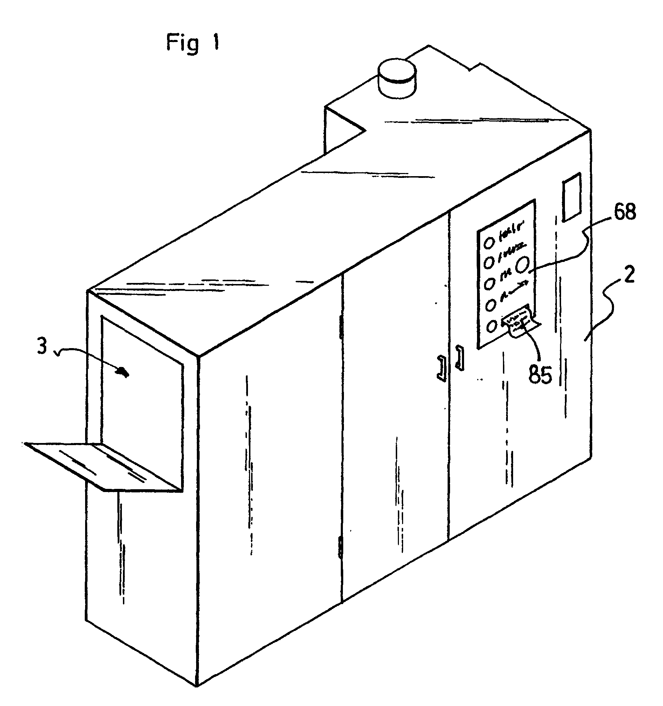

- la figure 1 est une vue schématique en perspective d'un appareil selon l'invention,
- la figure 2 est une vue schématique en coupe verticale longitudinale d'un appareil selon l'invention,
- les figures 3a, 3b, 3c, 3d sont des vues schématiques semblables à la figure 2 représentant plusieurs étapes successives d'un procédé selon l'invention,
- la figure 4 est une vue schématique en coupe verticale transversale par la chambre de broyage/stérilisation de l'appareil de la figure 2,

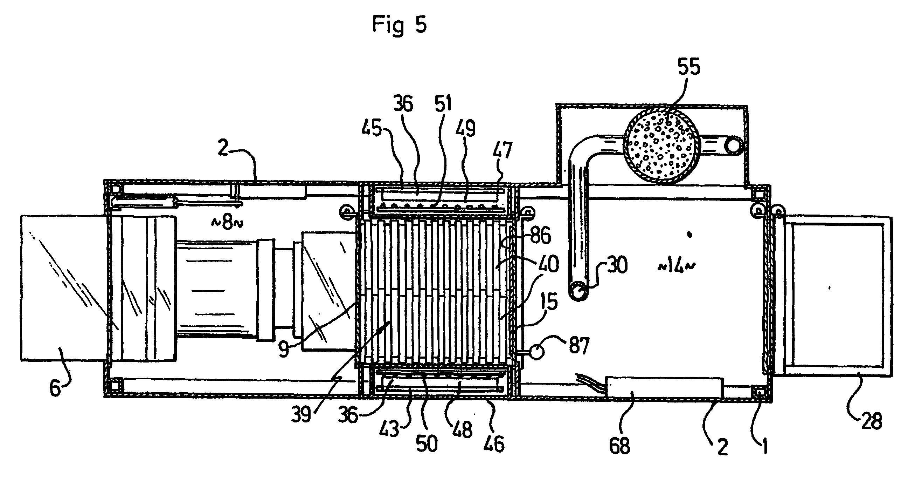

- la figure 5 est une vue schématique en coupe horizontale de l'appareil de la figure 2,
- la figure 6 est un schéma illustrant une variante de réalisation des moyens de chauffage et du poste de traitement des gaz d'un appareil selon l'invention.

L'appareil de traitement de déchets contaminés selon l'invention, représenté sur les figures, est en forme générale de caisson parallélépipédique défini par une structure de poutres métalliques formant un bâti 1 rigide, et de parois externes périphériques 2, de préférence métalliques, qui peuvent être garnies du côté intérieur d'une isolation thermique. Les parois externes 2 sont démontables ou peuvent être ouvertes pour accéder à l'intérieur de l'appareil.

L'appareil comprend une ouverture d'entrée 3 des déchets à traiter, dotée d'une trappe 4 articulée autour d'un axe horizontal et commandée par un vérin 5 entre une position ouverte et une position fermée. Un pan incliné 6 facilite l'introduction des déchets et leur alimentation dans la chambre 7 de broyage/stérilisation. L'ouverture d'entrée 3 donne accès à une première chambre 8 d'entrée qui est séparée de la chambre 7 de broyage/stérilisation par une paroi verticale 9, dotée d'une trappe 10 basculante par pivotement autour d'un axe 11 horizontal sous l'effet d'un vérin 12. La trappe 10 peut être placée soit en position ouverte où elle vient prolonger le pan incliné 6 vers le bas, une ouverture 13 étant laissée libre dans la paroi 9 pour l'introduction des déchets dans la chambre 7 de broyage/stérilisation, soit en position fermée où elle obture cette. ouverture 13 de façon hermétique. Si l'on souhaite renforcer la protection contre le risque de contamination des opérateurs, le pan incliné 6 peut, en variante non représentée, être omis. En effet, il ne peut être exclu que ce pan incliné 6 soit contaminé par le contact avec certains types de sacs non étanches. Les sacs de déchets sont alors directement placés sur la face supérieure de la trappe 10 qui est ensuite soumise au chauffage dans la chambre 7 de broyage/stérilisation et est donc aussi stérilisée.

La chambre 7 de broyage/stérilisation est séparée d'une chambre 14 de refroidissement, à l'opposé de la chambre d'entrée 8, par une paroi verticale 15 dans laquelle est ménagée une ouverture 16 refermée par une trappe 17 d'évacuation des déchets, montée pivotante autour d'un axe horizontal 18 et commandée par un vérin 19, de façon à basculer du côté intérieur de la chambre 7 de broyage/stérilisation.

La chambre 14 de refroidissement est délimitée, à l'opposé de la paroi 15 qui la sépare de la chambre 7 de broyage/stérilisation, par une paroi externe verticale 2 de l'appareil, dans laquelle une ouverture 20 de sortie des déchets traités est ménagée, cette ouverture 20 de sortie étant refermée par une trappe 21 articulée autour d'un axe horizontal commandée par un vérin 22.

La chambre 14 de refroidissement comprend également un plateau 23 monté pivotant autour d'un axe horizontal 24 et commandé par un vérin 25, de façon à pouvoir être placé soit contre un fond horizontal 26 de la chambre de refroidissement 14, sous l'ouverture 16 de la paroi 15, pour récupérer les déchets issus de la chambre 7 de broyage/stérilisation, soit dans une position d'éjection des déchets dans laquelle ce plateau 23 est relevé jusqu'à être incliné ou même vertical contre la paroi externe 2, de façon à faire tomber les déchets à travers l'ouverture 20 de sortie dans un sac de récupération 27 fixé sur une porte-sac 28 monté fixe sous l'ouverture de sortie 20.

La chambre 7 de broyage/stérilisation est aussi délimitée par une paroi verticale avant 46 qui peut être une portion de paroi externe 2, par une paroi verticale arrière 47 qui peut être une portion de paroi externe 2, par un plafond supérieur 29 refermant hermétiquement les parois verticales 9, 15, 46, 47, et par un fond inférieur 32 reliant hermétiquement les parties basses de ces parois 9, 15, 46, 47 verticales. Une conduite 30 d'évacuation des gaz débouche en haut de la chambre 7 de broyage/stérilisation. Une électrovanne 31 permet d'ouvrir ou de fermer cette conduite 30 d'évacuation des gaz. Un ventilateur ou compresseur 59 placé sur cette conduite 30 permet d'extraire les gaz de la chambre 7 de broyage/stérilisation.

Une conduite 33 débouche dans le fond 32 par l'intermédiaire d'un clapet anti-retour 34 pour l'alimentation en eau et/ou en vapeur d'eau dans la chambre 7 de broyage/stérilisation en cas de panne.

Ainsi, les parois 9, 15, 46; 47 de la chambre 7 de broyage/stérilisation définissent, lorsque les trappes 10, 17 sont fermées, une enceinte hermétiquement close, à l'exception de l'électrovanne 31 par laquelle les gaz de traitement peuvent être évacués. Le traitement de broyage et de stérilisation des déchets est effectué dans cette enceinte.

La chambre 7 de broyage/stérilisation renferme un tapis transporteur sans fin 35 à augets 36, guidé par des rouleaux de renvoi d'angle 37, dont l'un au moins est accouplé à un moteur 38. Les rouleaux 37 sont disposés de telle sorte que le tapis 35 défile en regard de la face interne de chacune des parois 46, 47, 29, 32, les augets 36 étant orientés vers l'intérieur. Le tapis définit ainsi une boucle sans fin le long des parois 46, 47, 29, 32, de la chambre 7 de broyage/stérilisation. A l'intérieur de cette boucle, la chambre 7 comprend un broyeur 39 disposé en partie inférieure et adapté pour recevoir les déchets à broyer par le haut, les déchets broyés étant dégagés vers le bas. Le broyeur 39 comprend une pluralité d'arbres de broyage 40 rotatifs entraînés en rotation par un groupe moteur 41. Le moteur 38 entraînant le tapis 35 et le groupe moteur 41 du broyeur 39 sont disposés à l'extérieur de la chambre 7 de broyage/stérilisation, de façon à ne pas subir les hautes températures qui y règnent au cours du traitement, et sont reliés aux rouleaux de renvoi 37, respectivement au broyeur 39, à travers une des parois de la chambre 7 de broyage/stérilisation, qui, dans l'exemple représenté, est la paroi 9 latérale verticale de séparation avec la chambre d'entrée 8. Au passage de la paroi 9, la liaison entre le moteur 38 et le rouleau 37 correspondant est adapté pour être hermétique, c'est-à-dire étanche aux gaz et aux liquides. De même, la liaison entre le groupe moteur 41 et le broyeur 39 au passage de la paroi 9 est adaptée pour être aussi hermétique.

Le tapis transporteur 35 peut, en variante non représentée, être remplacé par des moyens adaptés pour créer et entretenir un courant d'air pulsé à haute vitesse circulant sous le broyeur 39, puis de bas en haut dans une cheminée verticale latérale par laquelle les déchets remontent dans la chambre 7. A la sortie de cette cheminée, les déchets retombent dans la chambre 7 en passant en regard de moyens de chauffage jusqu'à l'entrée du broyeur 39. Ces moyens formant un courant d'air pulsé peuvent être formés d'une ou plusieurs buse(s) alimentée(s) par un ventilateur à haut débit placé dans la chambre 7, l'effet d'aspiration et d'entraînement étant éventuellement renforcé par un dispositif de Venturi.

Les différents vérins 5, 12, 19, 22, 25, 87 peuvent être des vérins pneumatiques ou électropneumatiques alimentés en air comprimé à partir d'un compresseur d'air électrique incorporé à l'appareil. Les vérins peuvent être remplacés par tous autres organes d'actionnement, par exemple des moteurs électriques. Ils sont placés en dehors de la chambre 7 de broage/stérilisation qui ne renferme aucun organe moteur ou d'actionnement sensible à la température.

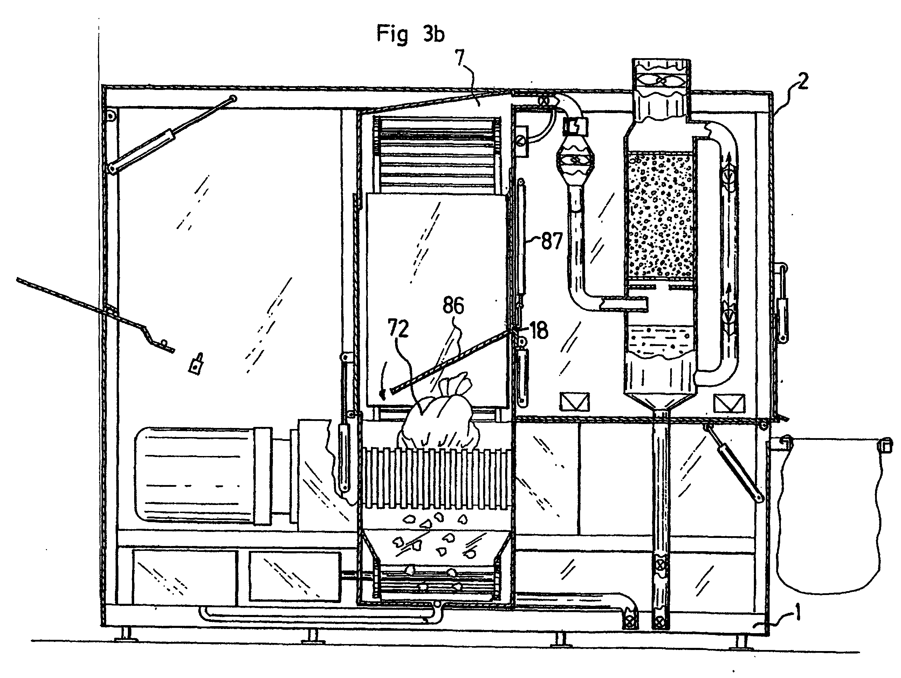

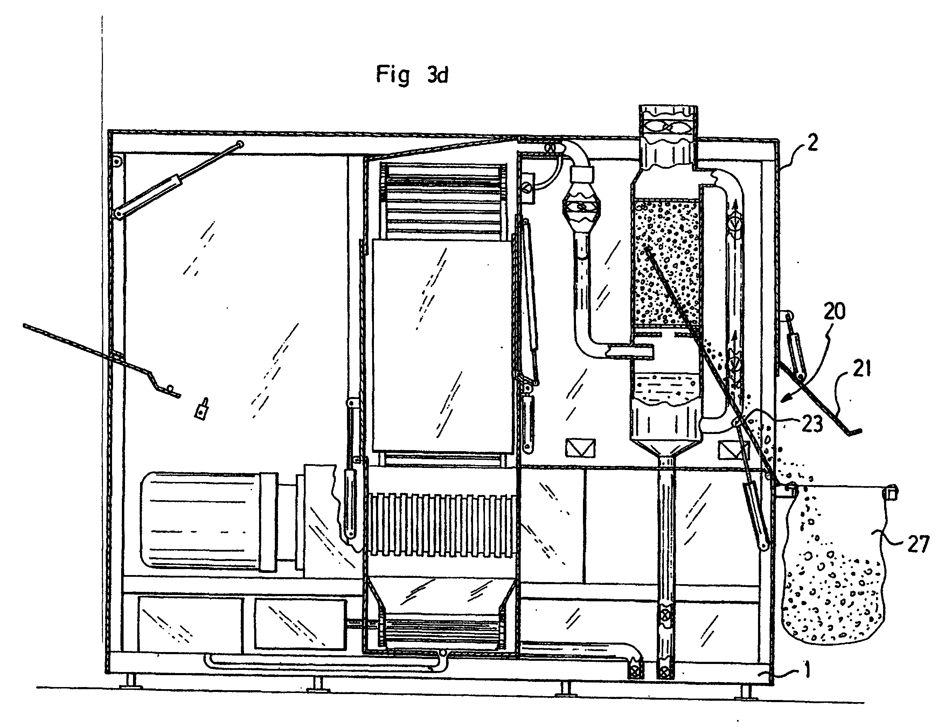

Avantageusement, un organe 86 d'appui des déchets sur le broyeur 39 est prévu pour exercer une pression sur le sac de déchets introduit dans la chambre 7 de broyage/stérilisation et forcer son passage à travers le broyeur 39. L'organe 86 d'appui peut aussi avoir pour fonction de briser ou déformer les déchets dont la forme nuirait à leur passage à travers le broyeur 39, tels que les bouteilles, atomiseurs, ... Cet organe 86 d'appui est articulé dans la chambre 7 de façon à pouvoir être replacé en position inactive dans laquelle il ne gêne pas la réintroduction des déchets dans le broyeur 39. L'organe 86 d'appui peut être formé d'une plaque, d'une grille, d'un ou plusieurs bras, ... Dans le mode de réalisation représenté, cet organe 86 d'appui est disposé sous la trappe 17 d'évacuation des déchets, articulé autour du même axe horizontal 18, et commandé par un vérin 87 disposé à l'extérieur de la chambre 7 dont la tige d'actionnement est reliée à une attache solidaire de l'organe 86 d'appui traversant une lumière ménagée dans la trappe 17 d'évacuation. Sur la figure 3b, l'organe 86 d'appui est représenté en position active où il force un sac de déchets à passer au travers du broyeur 39. Sur les autres figures, l'organe 86 d'appui est représenté en position inactive rappelé contre la trappe 17 d'évacuation, par exemple par un ressort (non représenté) intégré à l'axe 18 d'articulation commun à la trappe 17 et à l'organe 86 d'appui, le vérin 87 étant un vérin à simple effet. En variante, on peut aussi utiliser un vérin 87 à double effet commandé de façon appropriée, de façon à ne pas empêcher le fonctionnement du vérin 19 de la trappe 17 d'évacuation.

Le tapis 35 définit : un tronçon inférieur 42 s'étendant horizontalement sous la sortie du broyeur 39, les augets 36 étant orientés vers le haut pour récupérer les déchets broyés tombant du broyeur 39 ; un tronçon latéral remontant 43, s'étendant verticalement le long de la paroi avant 46 et passant dans un tunnel de chauffage 48 fixé à cette paroi avant 46 ; un tronçon supérieur 44 s'étendant horizontalement au-dessus du broyeur 39, les augets 36 étant orientés vers le bas, c'est-à-dire vers le broyeur 39 pour y décharger à nouveau les déchets ; et un tronçon latéral descendant 45 s'étendant verticalement le long de la paroi arrière 47 de la chambre 7 vers le bas, depuis le tronçon supérieur 44 jusqu'au tronçon inférieur 42. Le tronçon latéral descendant 45 passe également en regard d'un tunnel de chauffage 49.

Les tunnels de chauffage 48, 49 constituent des moyens de chauffage à sec, qui sont disposés dans la chambre 7 plus haut que le broyeur 39. Chaque tunnel de chauffage 48, 49 est adapté pour chauffer les déchets contenus dans les augets 36 et/ou ces augets 36 et le tapis 35 eux-mêmes par radiation et/ou par convection, à une température adaptée pour permettre la stérilisation des déchets et du tapis. Les deux tunnels 48, 49 sont avantageusement adaptés pour maintenir dans la chambre 7 une température au moins sensiblement homogène comprise entre 180°C et 220°C -notamment de l'ordre de 200°C-, apte à stériliser l'ensemble de la chambre 7 de broyage/stérilisation et les éléments qu'elle contient. Ainsi, les déchets présents dans la chambre 7 de broyage/stérilisation sont soumis à cette température et stérilisés sans interruption pendant toute la durée de leur présence dans la chambre 7. D'autres tunnels de chauffage peuvent être prévus dans la chambre 7, par exemple un tunnel de chauffage sous le tronçon inférieur 42 porté par le fond 32.

Chaque tunnel de chauffage 48, 49 porte des résistances électriques 50, 51 s'étendant en regard, du côté intérieur, du tapis 35 et de ses augets 36. Avantageusement, les résistances électriques 50, 51 sont portées par une paroi métallique verticale 52, respectivement 53, fixée à la paroi avant 46 ou arriére 47 de la chambre 7. Avantageusement, les résistances électriques sont disposées sur une face de cette paroi 52, 53 orientée vers les augets 36, de sorte que le chauffage ainsi réalisé est au moins en partie un chauffage par rayonnement.

Le tunnel de chauffage 48 de la paroi avant 46 permet ainsi de stériliser les déchets contenus dans les augets 36 du tronçon latéral remontant 43, c'est-à-dire les déchets issus du broyeur 39. Ces déchets sont ensuite recyclés à l'entrée du broyeur 39 lorsqu'ils arrivent au niveau du tronçon supérieur 44. Il est à noter que le tronçon supérieur 44 étant en partie haute de la chambre 7 alors que le broyeur 39 est en partie basse, les déchets chutent par gravité sur une certaine hauteur, ce qui a pour effet de les brasser et d'améliorer l'effet de la stérilisation par une meilleure homogénéisation et pénétration de la température au sein des déchets.

Le tunnel de chauffage 49 du tronçon latéral descendant 45 a pour effet de stériliser le tapis 35 et les augets 36 avant que ceux-ci ne récupèrent de nouveaux déchets éventuellement encore contaminés.

Le broyeur 39 peut être un broyeur de type traditionnel, à l'exception du fait qu'il est adapté pour pouvoir fonctionner à la température nominale de fonctionnement de la chambre 7 de broyage/stérilisation (comprise entre 180°C et 220°C, notamment de l'ordre de 200°C). Pour ce faire, il suffit par exemple de choisir de monter les arbres de broyage 40 avec un jeu suffisant pour tenir compte des dilatations thermiques, et d'utiliser un matériau résistant à cette température. Il est à noter en outre que le broyeur 39 étant incorporé à l'intérieur de la chambre 7 de broyage/stérilisation, et les déchets étant broyés selon plusieurs cycles de broyage successifs, le broyeur 39 peut présenter une finesse de broyage élémentaire relativement grossière pour chaque passe de broyage. Ainsi, un jeu relativement important peut être admis entre les différents couteaux de broyage. Ce jeu non seulement permet la prise en compte des dilatations thermiques, mais également évite le blocage du broyeur 39 et les incidents mécaniques dus au passage d'objets métalliques relativement durs dans le broyeur 39. En pratique, on constate qu'il est possible, avec un broyeur 39 présentant au moins une paire d'arbres de broyage 40, d'obtenir une taille moyenne des déchets broyés, après plusieurs cycles de broyage, inférieure à 10 mm, notamment de l'ordre de 2 mm à 5 mm.

Il est à noter que le broyeur 39 étant intégré au sein de la chambre 7 de broyage/stérilisation est lui-même soumis au chauffage de stérilisation en permanence. Plus exactement, toutes les pièces du broyeur 39 destinées à être au contact des déchets (notamment les rotors) sont disposées dans la chambre 7 de broyage/stérilisation, de sorte que ces pièces sont soumises à la stérilisation. Les opérations de maintenance ou de réparation sur ce broyeur 39 sont donc sans risque de contamination.

Une soupape de sécurité 54 est avantageusement prévue en partie haute d'une paroi de la chambre 7 pour empêcher toute augmentation intempestive de la pression dans cette chambre 7 à une valeur prédéterminée, par exemple de l'ordre de 1 à 2 bars (1 à 2.10⁵ Pa). Cette soupape 54 est disposée du côté de la chambre de refroidissement 14, sur la paroi verticale latérale 15, et reliée par une conduite à la conduite 30 d'évacuation des gaz, à l'aval de l'électrovanne 31.

Le fait que la chambre 7 soit hermétiquement close pendant le traitement permet d'éliminer les émissions gazeuses hors de l'appareil. Au cours du traitement, l'électrovanne 31 est ouverte, et on maintient la chambre 7 de broyage/stérilisation en légère dépression grâce à l'extraction et à l'évacuation permanente des gaz par le ventilateur 59.

En fin de traitement, la trappe 17 d'évacuation des déchets broyés bascule en position ouverte de façon à s'étendre sous le tronçon supérieur 44 du tapis transporteur 35, à l'intérieur de la boucle formée par ce tapis 35. De la sorte, les déchets broyés qui tombent du tronçon supérieur 44 sont captés par la trappe 17 d'évacuation lors de leur trajet vers le bas.

La conduite 30 d'évacuation appartient à un circuit d'évacuation des gaz de traitement comprenant un poste 55 de traitement des gaz avant leur rejet vers l'atmosphère. Ce poste 55 de traitement des gaz est en forme générale de colonne verticale et peut être disposé à l'intérieur de la chambre 14 de refroidissement tout en étant globalement isolé de l'atmosphère de la chambre 14 de refroidissement de façon à empêcher toute fuite intempestive de gaz depuis le circuit d'évacuation des gaz vers l'atmosphère interne de la chambre de refroidissement.

En outre, une isolation thermique est avantageusement prévue autour des parties du circuit d'évacuation des gaz en contact avec l'atmosphère de la chambre 14 de refroidissement (conduite 30, poste 55,...).

Des entrées 70 d'air frais extérieur de refroidissement sont prévues dans au moins une paroi 2 délimitant la chambre de refroidissement 14, de préférence sous la forme de clapets anti-retour empêchant la sortie de l'air par ces entrées 70. La conduite 30 d'évacuation est dotée d'au moins une bouche 71 d'extraction d'air par laquelle l'air de la chambre de refroidissement 14 est évacué dans le circuit d'évacuation, via le poste de traitement 55. La bouche 71 d'extraction est de préférence placée à l'amont du ventilateur ou compresseur 59 et peut être formée d'un clapet anti-retour empêchant tout retour de gaz depuis la conduite 30 dans la chambre de refroidissement 14.

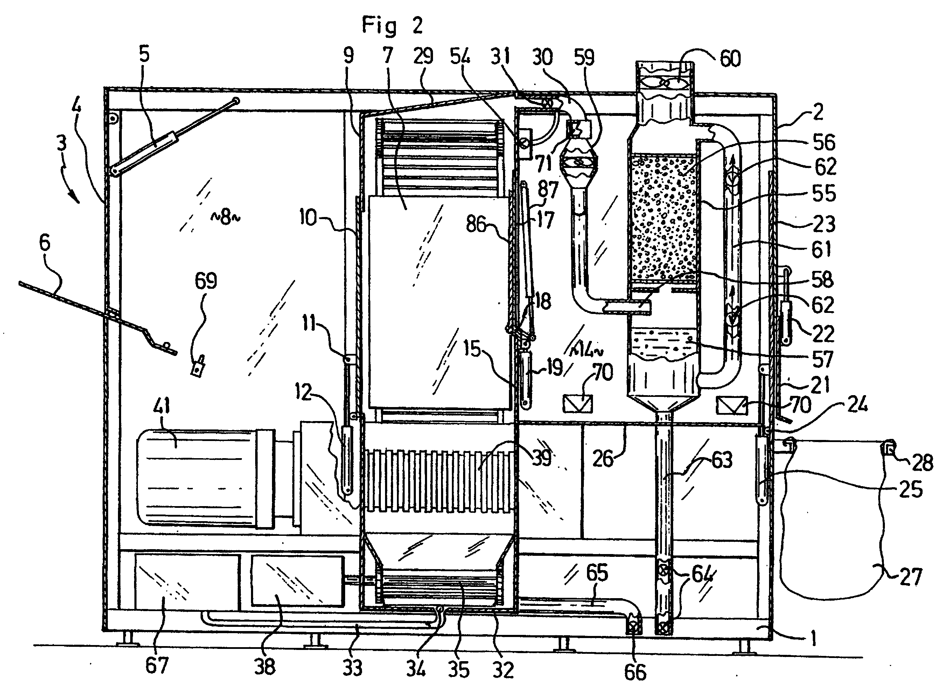

Le poste 55 de traitement des gaz comprend, dans le mode de réalisation représenté figure 2, une zone supérieure 56 garnie de matériaux adsorbants, une zone inférieure 57 comprenant une solution de chaux éteinte, et une arrivée des gaz à traiter 58 entre la zone supérieure 56 et la zone inférieure 57. Cette arrivée 58 est l'extrémité de la conduite 30 d'évacuation. Le ventilateur ou compresseur 59 de la conduite 30 permet d'aspirer et d'extraire les gaz depuis la chambre 7 de broyage/stérilisation jusqu'au poste 55 de traitement des gaz. Le poste 55 de traitement des gaz comprend à son extrémité supérieure ouverte également un ventilateur ou compresseur 60 qui souffle les gaz traités dans l'atmosphère, à travers un conduit de cheminée externe à l'appareil.

La zone inférieure 57 permet de traiter les gaz lourds qui sont récupérés après traitement par une conduite 61 reliant la partie basse de la zone inférieure 57 à la partie haute du poste de traitement 55 au-dessus de la zone supérieure 56 à l'amont du ventilateur ou compresseur 60. Au moins un clapet anti-retour 62 est prévu dans la conduite 61. La partie inférieure du poste 55 de traitement de gaz comprend une sortie d'évacuation des effluents liquides dans une conduite de vidange 63 dotée d'une ou plusieurs vannes 64.

Une conduite de vidange 65 est également prévue en partie inférieure de la chambre 7 de broyage/stérilisation pour vidanger cette chambre 7 en tant que de besoin. Cette conduite 65 de vidange est dotée d'une vanne de vidange 66.

La conduite 33 d'alimentation en eau de la chambre 7 de broyage/stérilisation en cas de panne est reliée à une source d'eau sous pression 67 qui est par exemple formée d'une réserve d'eau et d'une électropompe, ou d'une simple arrivée d'eau sous pression du réseau d'eau courante. En cas de panne de l'un des éléments de la chambre 7 de broyage/stérilisation (arrêt du défilement du tapis 35 ou arrêt du broyeur 39 ou arrêt du chauffage des tunnels 48, 49), une quantité d'eau est introduite à l'intérieur de la chambre 7 de broyage/stérilisation. Cette chambre 7 étant à haute température, cette eau s'évapore et stérilise l'ensemble de l'intérieur de la chambre 7. Une résistance électrique de secours (non représentée) à alimentation électrique est avantageusement prévue au fond 32 de la chambre 7 de broyage/stérilisation pour renforcer l'évaporation de l'eau en cas de panne des tunnels chauffants 48, 49 ou plus généralement au cas où la température ne serait pas suffisante. On peut ensuite procéder, d'une part, à la vidange de l'eau résiduelle par la conduite 65 de vidange, d'autre part, à l'intervention de réparation.

L'appareil comprend en outre un automate 68 qui est programmé pour automatiser tout ou partie du fonctionnement de l'appareil. Cet automate 68 peut être programmé pour contrôler le fonctionnement du broyeur 39 et/ou du tapis 35 et/ou des tunnels chauffants 48, 49 et/ou des différentes électrovannes et/ou des ventilateurs ou compresseurs 59, 60 et/ou des différents vérins d'actionnement des trappes et/ou de la source 67 d'eau sous pression...

Le procédé de traitement mis en oeuvre dans un tel appareil peut être le suivant.

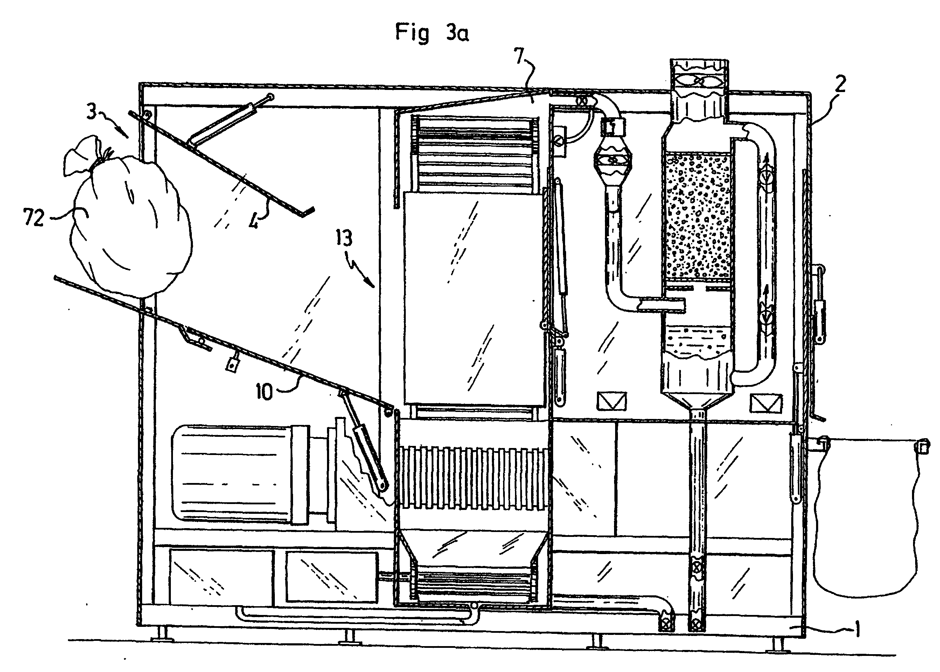

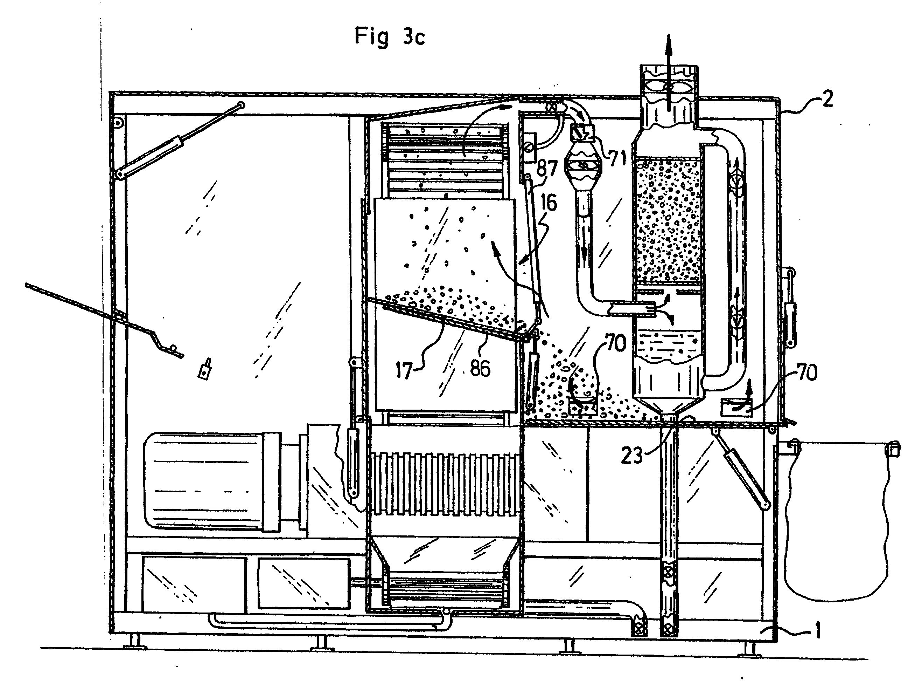

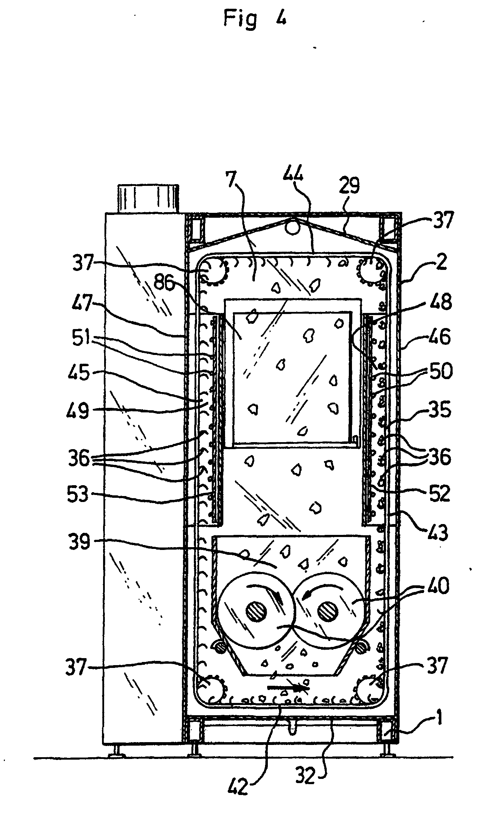

L'appareil est relié au réseau électrique et mis en tension, et, le cas échéant, au réseau d'eau courante. Les trappes 4, 10 d'entrée s'ouvrent. Le plateau 23 de la chambre de refroidissement 14 récupérant les déchets est placé à l'horizontale contre le fond 26 de la chambre 14 de refroidissement. Les tunnels chauffants 48, 49 sont alimentés en électricité pour être actifs en permanence et le broyeur 39 est mis en fonctionnement permanent, les rotors 40 étant entraînés en rotation permanente. Lors de l'introduction d'un sac 72 de déchets à stériliser sur le pan incliné 6 (figure 3a), celui-ci glisse vers le bas sur la trappe 10, et sa présence est détectée par un capteur 69 disposé sous cette trappe 10. Le tapis 35 de convoyage sans fin est alors mis en fonctionnement permanent à vitesse constante. Lorsque le sac arrive dans la chambre 7 de broyage/stérilisation au-dessus du broyeur 39, les trappes 4, 10 sont refermées, l'électrovanne 31 de la conduite d'évacuation des gaz 30 est ouverte, et la trappe 17 d'évacuation est fermée. La chambre 7 de broyage/stérilisation est alors hermétiquement close à l'exception de l'extraction des gaz par la conduite 30, les ventilateurs ou compresseurs 59, 60 étant en fonctionnement, et les déchets sont broyés dans le broyeur 39, puis recyclés et chauffés comme indiqué précédemment (figures 3b et 4). La température au sein de la chambre 7 augmente jusqu'à sa valeur nominale. La chambre 7 est en général en légère dépression. Le traitement est maintenu pendant une durée prédéterminée correspondant à une temporisation programmée de l'automate 68. La trappe 17 d'évacuation bascule en position ouverte (figure 3c), et les déchets broyés sont évacués sur le plateau 23 de la chambre de refroidissement 14. Il est à noter que simultanément, dans cette position, l'air frais arrive à l'intérieur de la chambre de refroidissement 14 par les entrées 70 et passe dans la chambre 7 de broyage/stérilisation à travers la trappe 17 ouverte, pour être ensuite évacué par l'intermédiaire de la conduite 30 d'évacuation et du poste 55 du traitement des gaz.

Le refroidissement des déchets broyés présents sur le plateau 23 s'effectue pendant une durée prédéterminée correspondant à une temporisation programmée de l'automate 68. Lorsque cette durée est atteinte, la trappe de sortie 21 est ouverte, et le plateau 23 est basculé vers le haut de façon à évacuer les déchets broyés, stérilisés et refroidis dans un sac 27 de sortie.

En cas de panne à l'intérieur de la chambre 7 de broyage/stérilisation au cours du traitement, de l'eau est introduite dans la chambre 7 de broyage/stérilisation grâce à la source 67 d'eau et à la conduite 33 d'alimentation. Cette eau est évaporée notamment grâce à la résistance de secours comme décrit précédemment. A la place de l'eau, on peut également prévoir d'introduire de la vapeur d'eau, si nécessaire.

Le dispositif porte-sac 28 est doté avantageusement de moyens de pesage, de moyens de soudage de l'ouverture du sac 27 de récupération des déchets traités, et de moyens 85 d'impression d'une étiquette permettant d'identifier notamment l'origine des déchets, les paramètres (température, durée...), la date et l'heure du traitement, tous ces moyens étant connus en eux-mêmes. Ces moyens 85 d'impression sont par exemple placés sur la face avant de l'appareil et contrôlés par l'automate 68 comprenant une horloge. interne qui fournit la date, l'heure et la durée du traitement. Au moins une sonde de température placée dans la chambre 7 de broyage/stérilisation fournit la température de traitement. L'étiquette est imprimée lorsque l'automate 68 déclenche l'ouverture de la trappe 21 de sortie.

Les essais permettent de démontrer que les déchets hospitaliers de diverses natures peuvent être traités, stérilisés et broyés et refroidis en une durée moyenne de l'ordre de 15 min dans un appareil selon l'invention. La consommation énergétique selon l'invention n'est pas excessive et le traitement effectué n'est pas polluant. Les seuls rejets sont les gaz traités qui ne sont pas polluants, et les éventuels effluents liquides du traitement des gaz lourds qui sont en volume très faible, voire inexistants dans la variante décrite ci-après.

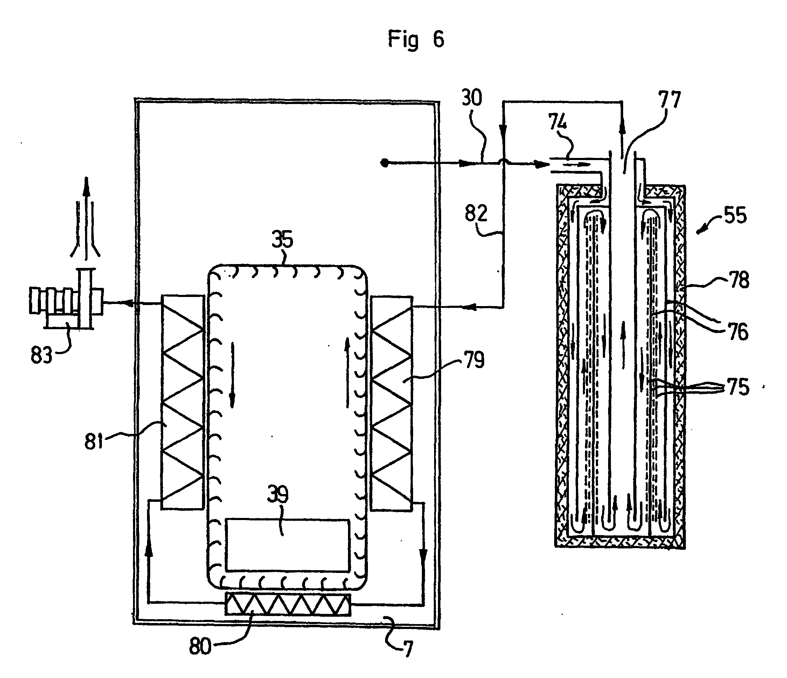

La figure 6 représente une variante de réalisation des moyens de chauffage de la chambre 7 de broyage/stérilisation et du poste 55 de traitement. Dans cette variante, les gaz évacués de la chambre 7 par la conduite 30 sont traités par le poste 55 à haute température (classiquement de l'ordre de 500 à 700°C). Ils subissent ainsi une combustion catalytique qui a pour effet de détruire par combustion les éventuels éléments polluants organiques qu'ils contiennent. Le poste 55 de traitement est alors formé d'une colonne renfermant une pluralité de cylindres 76 concentriques assemblés les uns aux autres de façon à former des chicanes de circulation des gaz jusqu'à ce qu'ils ressortent par l'intérieur du cylindre 77 de sortie de plus faible diamètre. L'un des cylindres internes 76 porte des résistances électriques 75 qui permettent de chauffer l'air arrivant par l'entrée 74, à la température souhaitée. L'ensemble est entouré d'une isolation thermique 78. En variante non représentée, ce poste de traitement 55 peut être lui-même incorporé dans la chambre 7 de broyage/stérilisation, notamment en partie haute, pour permettre la récupération de calories qui en sont issues.

La chambre 7 de broyage/stérilisation comprend par ailleurs des échangeurs thermiques 79, 80, 81 à l'intérieur desquels les gaz chauds issus du poste de traitement 55 sont amenés par une conduite 82. Les différents échangeurs 79, 80, 81 sont reliés les uns aux autres, et le dernier échangeur 81 a sa sortie reliée à un ventilateur d'extraction 83 qui rejette les gaz propres dans l'atmosphère.

Les échangeurs thermiques 79, 80, 81 sont des échangeurs à circuit de gaz séparé, les gaz circulant dans ces échangeurs ne se mélangeant pas avec l'atmosphère à l'intérieur de la chambre 7 de broyage/stérilisation. Toute forme d'échangeur thermique à circuit de gaz séparé peut être utilisée (serpentin, échangeur tubulaire ou à plaques...).

Les échangeurs thermiques 79, 80, 81 peuvent être prévus à la place des tunnels chauffants 48, 49 de la première variante, ou en combinaison avec ces tunnels.

Dans cette dernière variante, l'air est extrait en permanence de la chambre 7 de broyage/stérilisation par la conduite 30, l'électrovanne 31 étant ouverte pendant toute la durée du traitement. Cette extraction est effectuée par le ventilateur 83. Un autre ventilateur ou compresseur peut être prévu sur la conduite 30 comme dans la variante précédente et/ou sur la conduite 82 entre le poste de traitement 55 et les échangeurs thermiques 79, 80, 81.

A titre d'exemple, un appareil selon l'invention présente les caractéristiques suivantes : dimensions hors tout : longueur comprise entre 2m et 2,5m; hauteur comprise-entre 1,5m et 2m ; largeur comprise entre 0,5m et 1m; vitesse de défilement linéaire du tapis 35 : de 4 à 8m/min ; nombre de cycles de broyage : de 5 à 20 ; puissance électrique moyenne consommée : de 20 à 80 kW ; capacité de traitement : 30 à 60 kg/h.

L'invention peut faire l'objet de diverses autres variantes par rapport aux modes de réalisation décrits.

## Revendications

1. Procédé de traitement de déchets contaminés tels que des déchets hospitaliers dans lequel on traite les déchets dans une chambre (7) de broyage/stérilisation définissant une enceinte close au sein de laquelle on broie les déchets et on les stérilise par chauffage, **caractérisé en ce qu'**on fait circuler les déchets dans la chambre (7) de broyage/stérilisation en plusieurs cycles de broyage/stérilisation à travers des moyens (39) de broyage.

2. Procédé selon la revendication 1, **caractérisé en ce qu'**à chaque cycle de broyage, on soumet les déchets à un chauffage dans la chambre de (7) broyage/stérilisation à l'amont et/ou à l'aval et/ou au sein des moyens (39) de broyage.

3. Procédé selon l'une des revendications 1 et 2, **caractérisé en ce qu'**on stérilise les déchets par chauffage à sec -notamment par passage en regard de moyens (48, 49) de chauffage électrique et/ou en regard d'au moins un échangeur thermique (79, 80, 81)-.

4. Procédé selon l'une des revendications 1 à 3, **caractérisé en ce qu'**on fait circuler les déchets en continu en cycles successifs sans interruption à travers les moyens (39) de broyage entraînés en permanence et en regard de moyens (48, 49, 79, 80, 81) de chauffage maintenus actifs en permanence.

5. Procédé selon l'une des revendications 1 à 4, **caractérisé en ce qu'**on fait circuler les déchets en les récupérant en sortie des moyens (39) de broyage sur des moyens (35) de convoyage sans fin incorporés dans la chambre (7) de broyage/stérilisation et adaptés pour réintroduire à l'entrée des moyens (39) de broyage les déchets récupérés en sortie.

6. Procédé selon l'une des revendications 1 à 5, **caractérisé en ce que** pour chauffer les déchets, on place l'intérieur de la chambre (7) de broyage/stérilisation à une température apte à stériliser les déchets qui s'y trouvent, on maintient les déchets dans la chambre (7) de broyage/stérilisation pendant une durée adaptée pour assurer leur complète stérilisation **en ce qu'**on les transfère ensuite dans une chambre (14) de refroidissement séparée, et **en ce qu'**on introduit de nouveaux déchets dans la chambre (7) de broyage/stérilisation sans interruption des moyens (39) de broyage ni des moyens (48, 49, 79, 80, 81) de chauffage.

7. Procédé selon l'une des revendications 1 à 6, **caractérisé en ce qu'**on évacue les gaz de traitement vers un poste (55) de traitement des gaz avant leur rejet dans l'atmosphère.

8. Procédé selon la revendication 7, **caractérisé en ce qu'**on chauffe les gaz dans le poste (55) de traitement des gaz pour réaliser leur combustion catalytique.

9. Procédé selon l'une des revendications 7 ou 8, **caractérisé en ce qu'**on évacue les gaz de traitement hors de la chambre (7) de broyage/stérilisation en permanence pendant toute la durée du traitement, et **en ce qu'**on recycle les gaz chauds après leur traitement dans au moins un échangeur thermique (79, 80, 81) à circuit de gaz séparé disposé dans la chambre (7) de broyage/stérilisation.

10. Procédé selon l'une des revendications 1 à 9, **caractérisé en ce qu'**on maintient la chambre (7) de broyage/stérilisation au moins sensiblement hermétiquement close pendant toute la durée du traitement, à l'exception, le cas échéant, de l'évacuation des gaz de traitement.

11. Procédé selon l'une des revendications 7 à 10, **caractérisé en ce qu'**on maintient l'évacuation des gaz pendant le transfert des déchets vers la chambre (14) de refroidissement et pendant une durée supplémentaire adaptée pour assurer l'évacuation de l'ensemble des gaz avant d'introduire de nouveaux déchets à traiter dans la chambre (7) de broyage/stérilisation.

12. Procédé selon la revendication 5 et l'une des revendications 1 à 10, **caractérisé en ce qu'**en cas de panne des moyens (39) de broyage et/ou des moyens (48, 49, 79, 80, 81) de chauffage et/ou des moyens (35) de convoyage, on introduit dans la chambre (7) de broyage/stérilisation une quantité d'eau qui est évaporée par chauffage dans la chambre (7) de broyage/stérilisation et/ou une quantité de vapeur d'eau, cette quantité étant apte à stériliser l'ensemble des déchets et autres éléments présents dans la chambre (7) de broyage/stérilisation.

13. Procédé selon l'une des revendications 1 à 12, **caractérisé en ce qu'**on broie les déchets de façon à les diviser après plusieurs cycles de broyage en particules de moins de 10 mm de taille moyenne.

14. Procédé selon l'une des revendications 1 à 13, **caractérisé en ce qu'**on chauffe les déchets à une température comprise entre 180°C et 220°C.

15. Procédé selon l'une des revendications 1 à 14, **caractérisé en ce qu'**après refroidissement, on extrait puis on emballe les déchets traités, et on enregistre sur un support d'informations, des informations sur les déchets traités et/ou sur le traitement effectué.

16. Appareil de traitement de déchets contaminés tels que des déchets hospitaliers, comprenant une chambre (7) de broyage/stérilisation adaptée pour pouvoir définir une enceinte close renfermant des moyens (39) de broyage des déchets et des moyens (48, 49, 79, 80, 81) de chauffage adaptés pour pouvoir soumettre les déchets à un chauffage de façon à les stériliser, **caractérisé en ce que** la chambre (7) de broyage/stérilisation comporte des moyens (35) de convoyage adaptés pour pouvoir faire circuler les déchets en plusieurs cycles de broyage à travers les moyens (39) de broyage.

17. Appareil selon la revendication 16, **caractérisé en ce que** les moyens (35) de convoyage sont des moyens (35) de convoyage sans fin adaptés pour récupérer les déchets en sortie des moyens (39) de broyage et pour les réintroduire à l'entrée des moyens (39) de broyage.

18. Appareil selon l'une des revendications 16 ou 17, **caractérisé en ce que** les moyens (35) de convoyage sont adaptés pour transporter les déchets depuis la sortie des moyens (39) de broyage, puis en regard des moyens (48, 49, 79, 80, 81) de chauffage, puis à l'entrée des moyens (39) de broyage.

19. Appareil selon l'une des revendications 16 à 18, **caractérisé en ce que** les moyens (35) de convoyage sont adaptés pour passer sous les moyens (39) de broyage et récupérer les déchets par gravité en sortie des moyens (39) de broyage, pour remonter les déchets au-dessus des moyens (39) de broyage, et pour laisser ensuite retomber les déchets à l'entrée des moyens (39) de broyage par gravité.

20. Appareil selon l'une des revendications 16 à 19, **caractérisé en ce que** les moyens (35) de convoyage sont formés d'un tapis (35) transporteur sans fin à augets (36) passant autour et en dessous des moyens (39) de broyage et en regard des moyens (48, 49, 79, 80, 81) de chauffage.

21. Appareil selon l'une des revendications 16 à 19, **caractérisé en ce que** les moyens (35) de convoyage comportent des moyens pour créer et entretenir dans la chambre (7) de broyage/stérilisation un courant d'air pulsé à haute vitesse passant depuis la sortie des moyens de broyage puis circulant de bas en haut dans la chambre (7) de broyage/stérilisation pour y entraîner les déchets.

22. Appareil selon l'une des revendications 16 à 21, **caractérisé en ce que** les moyens de chauffage comportent des moyens (49, 81) de chauffage adaptés pour chauffer les déchets lors de leur descente et/ou pour renforcer la stérilisation des moyens (35) de convoyage après décharge des déchets et avant récupération à nouveau de déchets.

23. Appareil selon l'une des revendications 16 à 22, **caractérisé en ce que** les moyens (48, 49, 79, 80, 81) de chauffage sont des moyens de chauffage à sec -notamment électrique (48, 49) et/ou à échangeur(s) thermique(s) (79, 80, 81)-.

24. Appareil selon l'une des revendications 16 à 23, **caractérisé en ce qu'**il comprend une chambre (14) de refroidissement communiquant avec la chambre (7) de broyage/stérilisation par une porte (17).

25. Appareil selon la revendication 24, **caractérisé en ce que** la chambre (14) de refroidissement jouxte la chambre (7) de broyage/stérilisation, et **en ce que** la porte (17) est articulée de façon à former une trappe qui, en position ouverte, s'étend en regard et sous une portion (44) des moyens (35) de convoyage à partir de laquelle les déchets chutent vers l'entrée des moyens (39) de broyage, de sorte que cette porte (17) capte les déchets avant leur réintroduction à l'entrée des moyens (39) de broyage.

26. Appareil selon l'une des revendications 16 à 25, **caractérisé en ce qu'**il comprend un circuit (30, 55) d'évacuation des gaz comprenant un poste (55) de traitement des gaz avant leur rejet dans l'atmosphère.

27. Appareil selon les revendications 24 et 26, **caractérisé en ce que** le poste (55) de traitement des gaz est disposé dans la chambre (14) de refroidissement dont il est isolé.

28. Appareil selon l'une des revendications 26 ou 27, **caractérisé en ce que** le poste (55) de traitement comprend des moyens de chauffage des gaz aptes à réaliser leur combustion catalytique.

29. Appareil selon la revendication 28, **caractérisé en ce que** lesdits moyens de chauffage des gaz sont incorporés dans la chambre (7) de broyage/stérilisation.

30. Appareil selon l'une des revendications 26 à 29, **caractérisé en ce qu'**il comprend au moins un échangeur thermique (79, 80, 81) à circuit de gaz séparé, disposé dans la chambre (7) de broyage/stérilisation, et relié au poste (55) de traitement de gaz pour récupérer les gaz chauds après leur traitement, de sorte que cet échangeur thermique (79, 80, 81) constitue un moyen de chauffage de la chambre (7) de broyage/stérilisation et/ou des déchets en cours de traitement.

31. Appareil selon l'une des revendications 16 à 30, **caractérisé en ce que** la chambre (7) de broyage/stérilisation est adaptée pour pouvoir être hermétiquement close pendant toute la durée de traitement, à l'exception, le cas échéant, de l'évacuation des gaz de traitement.

32. Appareil selon l'une des revendications 16 à 31, **caractérisé en ce que** les moyens (39) de broyage sont adaptés pour broyer les déchets, de façon à les diviser, après plusieurs cycles de broyage, en particules de moins de 10 mm de taille moyenne.

33. Appareil selon l'une des revendications 16 à 32, **caractérisé en ce que** les moyens (48, 49, 79, 80, 81) de chauffage sont adaptés pour maintenir dans la chambre (7) de broyage/stérilisation une température comprise entre 180°C et 220°C.

34. Appareil selon l'une des revendications 16 à 33, **caractérisé en ce qu'**il comprend une alimentation (33, 67) en eau et/ou vapeur d'eau dans la chambre (7) de broyage/stérilisation permettant d'y introduire une quantité d'eau et/ou de vapeur d'eau, cette quantité étant apte à stériliser l'ensemble des déchets et autres éléments présents dans la chambre (7) de broyage/stérilisation en cas de panne des moyens (39) de broyage et/ou des moyens (48, 49, 79, 80, 81) de chauffage et/ou des moyens (35) de convoyage.

35. Appareil selon l'une des revendications 16 à 34, **caractérisé en ce qu'**il comprend des moyens (27, 28) d'emballage des déchets traités, et des moyens d'impression d'une étiquette présentant des informations sur les déchets traités et/ou le traitement effectué.

36. Appareil selon l'une des revendications 16 à 35, **caractérisé en ce qu'**il comprend un automate (68) programmable de contrôle.

## Claims

1. Method for treating contaminated waste, such as hospital waste, in which said waste is treated in a grinding/sterilisation chamber (7) defining a closed chamber inside which the waste is ground and sterilised by means of heating, **characterised in that** the waste is made to circulate inside the sterilisation/grinding chamber (7) in several grinding/sterilisation cycles through grinding means (39).

2. Method according to claim 1, **characterised in that** in each grinding cycle, the waste is subjected to heating inside the grinding/sterilisation chamber (7) upstream and/or downstream inside the grinding means (39).

3. Method according to claim 1 or 2, **characterised in that** the waste is sterilised by dry heating, especially via passage opposite electric heating means (48, 49) and/or opposite at least one heat exchanger (79, 80, 81).

4. Method according to one of claims 1 to 3, **characterised in that** the waste is made to circulate continuously uninterruptedly in successive cycles through the grinding means (39) driven continuously and opposite heating means (48, 49, 79, 80, 81) kept continuously active.

5. Method according to one of claims 1 to 4, **characterised in that** the waste is made to circulate by recovering it at the outlet of the grinding means (39) on endless transport means (35) incorporated in the grinding/sterilisation chamber (7) and adapted so as to re-introduce the waste recovered at the outlet into the inlet of the grinding means (39).

6. Method according to one of claims 1 to 5, **characterised in, that**, so as to heat the waste, the latter is placed inside the grinding/sterilisation chamber (7) at a temperature able to sterilise said waste located there, said waste being kept inside the grinding/sterilisation chamber (7) for a suitable period so as to ensure said waste is completely sterilised, **in that** said waste is then transferred into a separate cooling chamber (14), and **in that** new waste is introduced into the grinding/sterilisation chamber (7) without interruption from the grinding means (39) or from the heating means (48, 49, 79, 80, 81).

7. Method according to one of claims 1 to 6, **characterised in that** the treatment gases are removed to a gas treatment station (55) before being rejected into the atmosphere.

8. Method according to claim 7, **characterised in that** the gases are heated in the gas treatment station (55) so as to carry out their catalytic combustion.

9. Method according to claim 7 or 8, **characterised in that** the treatment gases are continuously removed from the grinding/sterilisation chamber (7) throughout the period of the treatment, and **in that** after being treated the hot gases are recycled in at least one heat exchanger (79, 80, 81) with a separate gas circuit placed in the grinding/sterilisation chamber (7).

10. Method according to one of claims 1 to 9,
**characterised in that** the grinding/sterilisation chamber (7) is kept at least approximately hermetically closed throughout the period of the treatment with the exception, if appropriate, of removal of the treatment gases.

11. Method according to one of claims 7 to 10, **characterised in that** removal of gases is maintained during the transfer of the waste to the cooling chamber (14) and during a suitable additional period to ensure removal of all the gases before introducing new waste to be treated inside the grinding/sterilisation chamber (7).

12. Method according to claim 5 and one of claims 1 to 10, **characterised in that** in the event of the breakdown of the grinding means (39) and/or the heating means (48, 49, 79, 80, 81) and/or the transport means (35), a quantity of water is introduced into the grinding/sterilisation chamber (7), said water being evaporated via heating inside the grinding/sterilisation chamber (7), and/or a quantity of water vapour, this quantity being suitable for sterilising all the waste and other elements present in the grinding/sterilisation chamber (7).

13. Method according to one of claims 1 to 12, **characterised in that** the waste is ground so as to divide them after several grinding cycles into particles smaller than 10 mm.

14. Method according to one of claims 1 to 13, **characterised in that** the waste is heated to a temperature of between 180°C and 220°C.

15. Method according to one of claims 1 to 14, **characterised that** after cooling the treated waste is then extracted and packed and recorded on an information medium, namely information concerning the treated waste and/or the treatment carried out.

16. Device for treating contaminated waste, such as hospital waste, said device including a suitable sterilisation/grinding chamber (7) so as to be able to define a closed chamber containing adapted waste grinding means (39) and heating means (48, 49, 79, 80, 81) adapted so as to be able to subject said waste to heating so as to sterilise it, **characterised in that** the grinding/sterilisation chamber (7) comprises suitable transport means (35) so as to be able to have the waste circulate in several grinding cycles through the grinding means (39).

17. Device according to claim 16, **characterised in that** the transport means (35) are adapted endless transport means (35) for recovering the waste at the outlet of the grinding means (39) and for introducing it at the inlet of the grinding means (39).

18. Device according to claim 16 or 17, **characterised in that** the transport means (35) are adapted to transport the waste from the outlet of the grinding means (39), then opposite the heating means (48, 49, 79, 80, 81) and then to the inlet of the grinding means (39).

19. Device according to one of claims 16 to 18, **characterised in that** the transport means (35) are adapted to have the waste pass under the grinding means (39) and recover the waste via gravity at the outlet of the grinding means (39), to lift said waste above the grinding means (39) and then allowing said waste to fall back to the inlet of the grinding means (39) via gravity.

20. Device according to one of claims 16 to 19, **characterised in that** the transport means (35) are formed of a an endless conveyor belt (35) with troughs (36) passing around and below the grinding means (39) and opposite the heating means (48, 49, 79, 80, 81).

21. Device according to one of claims 16 to 19, **characterised in that** the transport means (35) comprise means for creating and maintaining inside the grinding/sterilisation chamber (7) a high-speed pulsed current of air passing from the outlet of the grinding means and then circulating from bottom to top in the grinding/sterilisation chamber (7) so as to move the waste.

22. Device according to one of claims 16 to 21, **characterised in that** the heating means comprise adapted heating means (49, 81) for heating the waste at the time of their descent and/or for reinforcing sterilisation of the transport means (35) following discharging of the waste and before a further recovery of said waste.

23. Device according to one of claims 16 to 22, **characterised in that** the heating means (48, 49, 79, 80, 81) are dry heating means, especially electric (48, 49) and/or heat exchangers (79, 80, 81).

24. Device according to one of claims 16 to 23, **characterised in that** it includes a cooling chamber (14) communicating with the grinding/sterilisation, chamber (7) via a door (17).

25. Device according to claim 24, **characterised in that** the cooling chamber (14) is contiguous to the grinding/sterilisation chamber (7), and **in that** the door (17) is articulated so as to form a trap which, in an open position, extends opposite and under a portion (44) of the transport means (35) from which the waste falls towards the inlet of the grinding means (39) so that this door (17) captures the waste before the latter is reintroduced at the inlet of the grinding means (39).

26. Device according to one of claims 16 to 25, **characterised in that** it includes a gas evacuation circuit (30, 55) including a station (55) for treating the gases before they are rejected into the atmosphere.

27. Device according to claims 24 and 26, **characterised in that** the gas treatment station (55) is placed in the cooling chamber (14) from which it is isolated.

28. Device according to claim 26 or 27, **characterised in that** the treatment station (55) includes means for heating appropriate gases for embodying their catalytic combustion.

29. Device according to claim 28, **characterised in that** said gas heating means are incorporated in the grinding/sterilisation, chamber (7).

30. Device according to one of claims 26 to 29, **characterised in that** it includes at least one heat exchanger (79, 80, 81) with a separate gas circuit placed in the grinding/sterilisation chamber (7) and connected to the gas treatment station (55) so as to recover the hot gases after being treated so that this heat exchanger (79, 80, 81) constitutes an element for heating the grinding/sterilisation chamber (7) and/or the waste being treated.

31. Device according to one of claims 16 to 30, **characterised in that** the grinding/sterilisation chamber (7) is adapted so as to be hermetically sealed throughout the period of treatment, except, if appropriate, for evacuation of the treatment gases.

32. Device according to one of claims 16 to 31, **characterised in that** the grinding means (39) are adapted to grind the waste so as to divide it after several grinding cycles into particles smaller than 10 mm.

33. Device according to one of claims 16 to 32, **characterised in that** the heating means (48, 49, 79, 80, 81) are adapted so as to maintain a temperature of between 180°C and 220°C inside the grinding/sterilisation, chamber (7).

34. Device according to one of claims 16 to 33, **characterised in that** it includes a water and/or water vapour supply (33, 67) in the grinding/sterilisation chamber (7) making it possible to introduce a quantity or water and/or water vapour, said quantity being able to sterilise all the waste and other elements present in the grinding/sterilisation chamber (7) in the event of the malfunctioning of the grinding means (39) and/or the heating means (48, 49, 79, 80, 81) and/or the transport means (35).

35. Device according to one of claims 16 to 34, **characterised in that** it includes means (27, 28) for packing the treated waste and means for printing a label displaying information concerning the treated waste and/or the treatment carried out.

36. Device according to one of claims 16 to 35, **characterised in that** it includes a programmable control robot (68).

## Patentansprüche

1. Verfahren zur Behandlung von verunreinigten Abfällen, wie zum Beispiel Krankenhausabfälle, bei dem man die Abfälle in einer Zerkleinerungs-/Sterilisationskammer (7) behandelt, die eine geschlossene Einfassung definiert, innerhalb derer man die Abfälle zerkleinert und man sie durch Erhitzen sterilisiert, **dadurch gekennzeichnet, dass** man die Abfälle in der Zerkleinerungs/Sterilisationskammer (7) in mehreren Zerkleinerungs- / Sterilisationszyklen durch Zerkleinerungsmittel (39) umlaufen lässt.

2. Verfahren gemäß Anspruch 1, **dadurch gekennzeichnet, dass** man bei jedem Zerkleinerungszyklus die Abfälle einem Erhitzen in der Zerkleinerungs-/Sterilisationskammer (7) oberhalb und/oder unterhalb und/oder innerhalb der Zerkleinerungsmittel (39) aussetzt.

3. Verfahren gemäß einem der Ansprüche 1 und 2, **dadurch gekennzeichnet**, das man die Abfälle durch trockenes Erhitzen - insbesondere durch einen Durchgang gegenüber von elektrischen Heizmitteln (48, 49) und/oder gegenüber von wenigstens einem Wärmeaustauscher (79, 80, 81) sterilisiert.

4. Verfahren gemäß einem der Ansprüche 1 bis 3, **dadurch gekennzeichnet, dass** man die Abfälle im Durchlaufverfahren in aufeinander folgenden Zyklen ohne Unterbrechung durch die permanent angetriebenen und gegenüber den permanent aktiv gehaltenen Erhitzungsmitteln (48, 49, 79, 80, 81) liegenden Zerkleinerungsmittel (39) durchlaufen lässt.

5. Verfahren gemäß einem der Ansprüche 1 bis 4, **dadurch gekennzeichnet, dass** man die Abfälle durch Auffangen am Ausgang der Zerkleinerungsmittel (39) auf in die Zerkleinerungs-/Sterilisationskammer (7) integrierte und zum Wiedereinführen der am Ausgang aufgefangenen Abfälle am Eingang der Zerkleinerungsmittel (39) angepasste Endlos-Beförderungsmitteln (35) durchlaufen lässt.

6. Verfahren gemäß einem der Ansprüche 1 bis 5, **dadurch gekennzeichnet, dass** man zum Erhitzen der Abfälle das Innere der Zerkleinerungs-/Sterilisationskammer (7) einer Temperatur aussetzt, die zum Sterilisieren der sich dort befindlichen Abfälle geeignet ist, man die Abfälle während einer für die Gewährleistung ihrer vollständigen Sterilisation angepassten Zeitdauer in der Zerkleinerungs/Sterilisationskammer (7) behält, dass man sie anschließend in eine separate Kühlkammer (14) transferiert, und dass man ohne Unterbrechung der Zerkleinerungsmittel (39) noch der Erhitzungsmittel (48, 49, 79, 80, 81) neue Abfälle in die Zerkleinerungs-/Sterilisationskammer (7) einführt.

7. Verfahren gemäß einem der Ansprüche 1 bis 6, **dadurch gekennzeichnet, dass** man die Behandlungsgase vor ihrer Abgabe in die Atmosphäre an einen Behandlungsposten (55) der Gase austrägt.

8. Verfahren gemäß Anspruch 7, **dadurch gekennzeichnet, dass** man die Gase im Behandlungsposten (55) der Gase erhitzt, um ihre katalytische Verbrennung durchzuführen.

9. Verfahren gemäß Anspruch 7 oder 8, **dadurch gekennzeichnet, dass** man die Behandlungsgase außerhalb der Zerkleinerungs-/Sterilisationskammer (7) während der gesamten Dauer der Behandlung permanent austrägt und dass man die heißen Gase nach ihrer Behandlung in wenigstens einem Wärmeaustauscher (79, 80, 81) mit separatem, in der Zerkleinerungs-/Sterilisationskammer (7) angeordnetem Gaskreislauf wiederaufbereitet.

10. Verfahren gemäß einem der Ansprüche 1 bis 9, **dadurch gekennzeichnet, dass** man die Zerkleinerungs/Sterilisationskammer (7) wenigstens während der gesamten Dauer der Behandlung deutlich hermetisch geschlossen hält, ausgenommen eventuell den Austrag der Behandlungsgase.

11. Verfahren gemäß einem der Ansprüche 7 bis 10, **dadurch gekennzeichnet, dass** man den Austrag der Gase während des Transfers der Abfälle zur Kühlkammer (14) und während einer zusätzlichen Dauer aufrecht erhält, die angepasst ist, um den Austrag der gesamten Gase vor der Einführung neuer in der Zerkleinerungs-/Sterilisationskammer (7) zu behandelnder Abfälle zu gewährleisten.

12. Verfahren gemäß einem der Ansprüche 5 und Anspruch 1 bis 10, **dadurch gekennzeichnet, dass** man bei einem Ausfall der Zerkleinerungsmittel (39) und/oder der Erhitzungsmittel (48, 49, 79, 80, 81) und/oder der Beförderungsmittel (35) in die Zerkleinerungs/Sterilisationskammer (7) eine Wassermenge einführt, die durch Erhitzen in der Zerkleinerungs-/Sterilisationskammer (7) verdampft wird, und/oder eine Menge Wasserdampf, wobei diese Menge zur Sterilisation der gesamten Abfälle und anderer in der Zerkleinerungs-/Sterilisationskammer (7) vorhandener Elemente geeignet ist.

13. Verfahren gemäß einem der Ansprüche 1 bis 12, **dadurch gekennzeichnet, dass** man die Abfälle derart zerkleinert, dass sie nach mehreren Zerkleinerungszyklen in Partikel von weniger als 10 mm durchschnittlicher Größe geteilt werden.

14. Verfahren gemäß einem der Ansprüche 1 bis 13, **dadurch gekennzeichnet, dass** man die Abfälle auf eine zwischen 180° C und 200° C inbegriffene Temperatur erhitzt.

15. Verfahren gemäß einem der Ansprüche 1 bis 14, **dadurch gekennzeichnet, dass** man nach dem Abkühlen die behandelten Abfälle herausnimmt und dann verpackt und man auf einem Informationsträger Informationen über die behandelten Abfälle und/oder über die erfolgte Behandlung einträgt.

16. Vorrichtung zur Behandlung von verunreinigten Abfällen, wie zum Beispiel Krankenhausabfälle, umfassend eine Zerkleinerungs-/Sterilisationskammer (7), die angepasst ist, um eine geschlossene Einfassung (39) zu definieren, die Zerkleinerungsmittel (39) der Abfälle und Erhitzungsmittel (48, 49, 79, 80, 81) umschließt, die angepasst sind, um die Abfälle einer Erhitzung zu ihrer Sterilisation unterziehen zu können, **dadurch gekennzeichnet, dass** die Zerkleinerungs-/Sterilisationskammer (7) Beförderungsmittel (35) umfasst, die angepasst sind, um die Abfälle in mehreren Zerkleinerungszyklen durch die Zerkleinerungsmittel (39) durchlaufen zu lassen.

17. Vorrichtung gemäß Anspruch 16, **dadurch gekennzeichnet, dass** die Beförderungsmittel (35) Endlos-Beförderungsmittel (35) sind, die angepasst sind, um die Abfälle am Ausgang der Zerkleinerungsmittel (39) aufzufangen und um sie am Eingang der Zerkleinerungsmittel (39) wieder zuzuführen.

18. Vorrichtung gemäß Anspruch 16 oder 17, **dadurch gekennzeichnet, dass** die Beförderungsmittel (35) angepasst sind, um die Abfälle ab dem Ausgang der Zerkleinerungsmittel (39), dann gegenüber den Erhitzungsmitteln (48, 49, 79, 80, 81) und dann zum Eingang der Zerkleinerungsmittel (39) zu transportieren.

19. Vorrichtung gemäß einem der Ansprüche 16 bis 18, **dadurch gekennzeichnet, dass** die Beförderungsmittel (35) angepasst sind, um unter den Zerkleinerungsmitteln (39) hindurchzutreten und die Abfälle am Ausgang der Zerkleinerungsmittel (39) durch Schwerkraft aufzufangen, um die Abfälle über die Zerkleinerungsmittel (39) wieder heraufzuheben und um anschließend die Abfälle am Eingang der Zerkleinerungsmittel (39) durch Schwerkraft wieder fallen zu lassen.

20. Vorrichtung gemäß. einem der Ansprüche 16 bis 19, **dadurch gekennzeichnet, dass** die Beförderungsmittel (35) aus einem Endlos-Transportband (35) mit um die Zerkleinerungsmittel (39) und unter ihnen und gegenüber den Erhitzungsmitteln (48, 49, 79, 80, 81) durchtretenden Bechern (36) gebildet werden.

21. Vorrichtung gemäß einem der Ansprüche 16 bis 19, **dadurch gekennzeichnet, dass** die Beförderungsmittel (35) Mittel zur Schaffung und Aufrechterhaltung eines mit hoher Geschwindigkeit in der Zerkleinerungs/Sterilisationskammer (7) ab dem Ausgang der Zerkleinerungsmittel pulsierenden, anschließend von unten nach oben in der Zerkleinerungs-/Sterilisationskammer (7) zirkulierenden Luftstrom umfassen, um die Abfälle dorthin zu verbringen.

22. Vorrichtung gemäß einem der Ansprüche 16 bis 21, **dadurch gekennzeichnet, dass** die Erhitzungsmittel Erhitzungsmittel (49, 81) umfassen, die angepasst sind, um die Abfälle bei ihrem Abstieg zu erhitzen und/oder um die Sterilisation der Beförderungsmittel (35) nach dem Abladen der Abfälle und vor dem erneuten Aufnehmen von Abfällen zu verstärken.

23. Vorrichtung gemäß einem der Ansprüche 16 bis 22, **dadurch gekennzeichnet, dass** die Erhitzungsmittel (48, 49, 79, 80, 81) Mittel zur trockenen- insbesondere elektrischen (48, 49) - Erhitzung - und/oder mit Wärmeaustauscher(n) (79, 80, 81) sind.

24. Vorrichtung gemäß einem der Ansprüche 16 bis 23, **dadurch gekennzeichnet, dass** sie eine mit der Zerkleinerungs-/Sterilisationskammer (7) durch eine Tür (17) verbundene Kühlkammer (14) umfasst.

25. Vorrichtung gemäß Anspruch 24, **dadurch gekennzeichnet, dass** die Kühlkammer (14) neben der Zerkleinerungs-/Sterilisationskammer (7) liegt und dass die Tür (17) derart artikuliert ist, dass eine Klappe gebildet wird, die sich in geöffneter Position gegenüber und unter einem Abschnitt (44) der Beförderungsmittel (35) erstreckt, von dem aus die Abfälle zum Eingang der Zerkleinerungsmittel (39) derart fallen, dass diese Tür (17) die Abfälle vor ihrer erneuten Zuführung am Eingang der Zerkleinerungsmittel (39) auffängt.

26. Vorrichtung gemäß einem der Ansprüche 16 bis 25, **dadurch gekennzeichnet, dass** sie einen Austragskreislauf (30, 55) der Gase. mit einem Behandlungsposten (55) der Gase vor ihrer Abgabe in die Atmosphäre umfasst..

27. Vorrichtung gemäß Ansprüche 24 und 26, **dadurch gekennzeichnet, dass** der Behandlungsposten (55) der Gase in der Kühlkammer (14) angeordnet ist, von der er isoliert ist.

28. Vorrichtung gemäß Anspruch 26 oder 27, **dadurch gekennzeichnet, dass** der Behandlungsposten (55) Erhitzungsmittel der Gase umfasst, die geeignet sind, deren katalytische Verbrennung zu realisieren.

29. Vorrichtung gemäß Anspruch 28, **dadurch gekennzeichnet, dass** die besagten Erhitzungsmittel der Gase in die Zerkleinerungs-/Sterilisationskammer (7) integriert sind.

30. Vorrichtung gemäß einem der Ansprüche 26 bis 29, **dadurch gekennzeichnet, dass** sie wenigstens einen Wärmeaustauscher (79, 80, 81) mit separatem, in der Zerkleinerungs-/Sterilisationskammer (7) angeordnetem und mit dem Behandlungsposten (55) für Gase verbundenen Gaskreislauf umfasst, um die heißen Gase nach ihrer Behandlung aufzufangen, so dass dieser Wärmeaustauscher (79, 80, 81) ein Erhitzungsmittel der Zerkleinerungs-/Sterilisationskammer (7) und/oder der in der Behandlung befindlichen Abfälle bildet.

31. Vorrichtung gemäß einem der Ansprüche 16 bis 30, **dadurch gekennzeichnet, dass** die Zerkleinerungs/Sterilisationskammer (7) angepasst ist, um während der gesamten Dauer der Behandlung hermetisch geschlossen zu sein, ausgenommen beim Austrag der Behandlungsgase ggf.

32. Vorrichtung gemäß einem der Ansprüche 16 bis 31, **dadurch gekennzeichnet, dass** die Zerkleinerungsmittel (39) angepasst sind, um die Abfälle zu zerkleinern, s.o dass sie nach mehreren Zerkleinerungszyklen in Partikel einer Größe von weniger als 10 mm Durchschnittsgröße zerteilt werden.

33. Vorrichtung gemäß einem der Ansprüche 16 bis 32, **dadurch gekennzeichnet, dass** die Erhitzungsmittel (48, 49, 79, 80, 81) angepasst sind, um in der Zerkleinerungs/Sterilisationskammer (7) eine zwischen 180° C und 220° C inbegriffene Temperatur aufrecht zu erhalten.

34. Vorrichtung gemäß einem der Ansprüche 16 bis 33, **dadurch gekennzeichnet, dass** sie eine Wasser- und/oder Wasserdampfversorgung (33, 67) in der Zerkleinerungs/Sterilisationskammer (7) umfasst, die das Zuführen einer Wassermenge und/oder einer Menge Wasserdampf erlaubt, wobei diese Menge geeignet ist, die gesamten Abfälle und anderen, in der Zerkleinerungs-/Sterilisationskammer (7) vorhandenen Abfälle bei einem Ausfall der Zerkleinerungsmittel (39) und/oder der Erhitzungsmittel (48, 49, 79, 80, 81) und/oder der Beförderungsmittel (35) zu sterilisieren.

35. Vorrichtung gemäß einem der Ansprüche 16 bis 34, **dadurch gekennzeichnet, dass** sie Verpackungsmittel (27, 28) der behandelten Abfälle und Druckmittel eines Informationen über die behandelten Abfälle und/oder die ausgeführte Behandlung aufweisenden Etiketts umfasst.

36. Vorrichtung gemäß einem der Ansprüche 16 bis 35, **dadurch gekennzeichnet, dass** sie einen programmierbaren Kontrollautomaten (68) umfasst.
